# EUROPEAN PATENT APPLICATION

(11) **EP 4 427 756 A1**
(43) Date of publication of application: **11.09.2024**
(21) Application number: 22889986.0
(22) Date of filing: 02.11.2022
(51) Int. Cl.: A61K 35/744, A61K 36/06, A61P 3/10, A61P 43/00

(54) **DPP-4 INHIBITOR AND PRODUCTION METHOD THEREFOR**

(30) Priority: 05.11.2021 JP 2021181022
(71) Applicant: Sumitomo Chemical Company, Limited, Tokyo 103-6020 (JP)
(72) Inventor: KUSUMOTO, Masanori, Osaka-shi, Osaka 554-8558 (JP); YONEYAMA, Toshihiro, Osaka-shi, Osaka 554-8558 (JP); AOKI, Mikio, Osaka-shi, Osaka 554-8558 (JP); MIKATA, Kazuki, Osaka-shi, Osaka 554-8558 (JP)
(74) Representative: Hoffmann Eitle
(86) International application number: PCT/JP2022/041030
(87) International publication number: WO 2023/080166

(57) **Abstract**

An aspect of the present invention is directed to provide a novel dipeptidyl peptidase IV (DPP-4) inhibitor and a production method thereof.

The DPP-4 inhibitor according to an aspect of the present invention contains a hydrolysate obtained by hydrolyzing a protein derived from a microorganism. An article according to another aspect of the present invention for preventing and/or improving abnormality involving DPP-4 contains the DPP-4 inhibitor. A method for producing the DPP-4 inhibitor according to another aspect of the present invention includes a step of hydrolyzing a protein derived from a microorganism with a protease.

## Description

### Technical Field

An aspect of the present invention relates to a dipeptidyl peptidase IV (DPP-4) inhibitor and a method for producing the inhibitor.

### Background Art

It is known that when a hyperglycemic state is continued, for example, by diabetes continues, various complications occur. Insulin is the only one hormone that lowers blood glucose levels and its secretion is promoted by an incretin. GLP-1 is a kind of incretin and decomposed by dipeptidyl peptidase IV (DPP-4). Accordingly, a DPP-4 inhibitor has an action to lower blood glucose levels.

In connection with this, it has been reported that a hydrolysate of a protein exhibits DPP-4 inhibitory activity (see, for example, Patent Literatures 1 to 4).

### Citation List

### Patent Literature

Patent Literature 1: Japanese Patent Laid-Open No. 2013-040111
Patent Literature 2: International Publication No. WO 2012/102308
Patent Literature 3: International Publication No. WO 2014/199780
Patent Literature 4: International Publication No. WO 2006/068480

### Summary of Invention

### Problems to be Solved by Invention

Even if research has been conducted as disclosed in the prior-art documents mentioned above, the research for DPP-4 inhibitors has not yet been sufficient. An aspect of the present invention is directed to providing a novel DPP-4 inhibitor using a microorganism capable of easily proliferating as a protein source and a method for producing the DPP-4 inhibitor.

### Means to Solve the Problems

The dipeptidyl peptidase IV (DPP-4) inhibitor according to an aspect of the present invention contains a hydrolysate obtained by hydrolyzing a protein derived from a microorganism with a protease.

A method for producing a dipeptidyl peptidase IV (DPP-4) inhibitor according to an aspect of the present invention includes a step of hydrolyzing a protein derived from a microorganism with a protease.

### Effects of Invention

According to an aspect of the present invention, there are provided a novel DPP-4 inhibitor and a method for producing the inhibitor.

### Brief Description of Drawings

[Figure 1] Figure 1 shows bar charts showing the DPP-4 inhibitory activities of hydrolysates obtained by hydrolyzing 4 types of microorganisms (beer yeast, torula yeast, baker's yeast and lactic acid bacterium (*Lactobacillus plantarum*)) with various types of proteases.
[Figure 2] Figure 2 shows bar charts showing the DPP-4 inhibitory activities of hydrolysates obtained by hydrolyzing 3 types of lactic acid bacteria (*Lactococcus lactis, Enterococcus faecalis* and *Lactobacillus sakei*) with 2 types of proteases. In the figure, "B1" represents PROTIN SD-AY10 and "N6" represents Sumizyme LP-G.

### Embodiments for Carrying out Invention

Now, the embodiments of the present invention will be more specifically described but the aspects of the present invention are not limited to these. In the specification, unless otherwise specified, "A to B" representing a numerical range means "A or more to B or less". In the specification, amino acids are represented by single alphabetic letters according to the notation commonly known to those skilled in the art.

### [1. Method for producing DPP-4 inhibitor]

A method for producing a DPP-4 inhibitor according to an aspect of the present invention includes a step of hydrolyzing a protein derived from a microorganism with a protease. It was not previously known that a hydrolysate of a protein derived from a microorganism exhibits DPP-4 inhibitory activity. The protein derived from a microorganism is easily produced in a large amount and supplied stably. It is also expected to reduce its production cost.

### [1.1. Protein derived from microorganism]

Examples of a protein derived from a microorganism include microbial cells, microbial extracts, and proteins secreted by microorganisms outside the cells. For the reason that handling is easy and a large amount of protein is prepared, microbial cells are preferably used as a protein derived from a microorganism.

Examples of microorganisms include fungi and bacteria. Examples of the fungi include yeasts, molds and mushrooms. Examples of the bacteria include lactic acid bacteria, *Escherichia coli* bacteria, bacteria of the genus *Bacillus,* bifidobacteria and butyric acid bacteria. In view of easy-to-proliferate and prepare a large amount of microbial cells, yeasts or lactic acid bacteria are

### preferably used as a material.

Examples of the yeasts include fungi belonging to the genera *Saccharomyces, ShizoSaccharomyces, Cyberlindnera* and *Candida.* Examples of the yeasts depending on the use include baker's yeast, beer yeast, shochu yeast, sake yeast, wine yeast and torula yeast.

Examples of the lactic acid bacteria include bacteria belonging to the genera *Lactobacillus, Enterococcus, Lactococcus, Streptococcus, Bifidobacterium, Leuconostoc, Pediococcus* and *Wissella.* Of these, a bacteria of the genus *Lactobacillus* is preferable.

Examples of the bacteria of the genus *Lactobacillus* include *Lactobacillus casei, Lactobacillus crispatus, Lactobacillus acidophilus, Lactobacillus delbrueckii* subsp. *bulgaricus, Lactobacillus helveticus, Lactobacillus delbrueckii* subsp. *lactis, Lactobacillus plantarum, Lactobacillus gasseri, Lactobacillus acidipiscis, Lactobacillus brevis, Lactobacillus coryniformis, Lactobacillus delbrueckii* subsp. *delbrueckii, Lactobacillus insicii, Lactobacillus kefiri, Lactobacillus kefiranofaciens* subsp. *kefiranofaciens, Lactobacillus kefiranofaciens* subsp. *kefirgranum, Lactobacillus nodensis, Lactobacillus parabrevis, Lactobacillus paracasei, Lactobacillus parakefiri, Lactobacillus pentosus, Lactobacillus perolens, Lactobacillus rhamnosus, Lactobacillus sakei, Lactobacillussalivarius, Lactobacillus tucceti, Lactobacillus curvatus, Lactobacillus johnsonii, Lactobacillus fermentum* and *Lactobacillus mali.*

Examples of the *Enterococcus* bacteria include *Enterococcus faecalis, Enterococcus faecium, Enterococcus avium, Enterococcus gallinarum, Enterococcus casseliflavus* and *Enterococcus gallinarum.*

Examples of the *Lactococcus* bacteria include *Lactococcus lactis, Lactococcus lactis* subsp. *lactis, Lactococcus lactis* subsp. *cremoris, Lactococcus lactis* subsp. *lactis biovar. diacetylactis* and *Lactococcus raffinolactis.*

Examples of the *Streptococcus* bacteria include *Streptococcus thermophilus, Streptococcus mutans, Streptococcus faecalis* and *Streptococcus agalactiae.*

Examples of the *Bifidobacterium* bacteria include *Bifidobacterium infantis, Bifidobacterium adolescentis, Bifidobacterium breve, Bifidobacterium bifidum, Bifidobacterium longum, Bifidobacterium longum* subsp. *longum, Bifidobacterium pseudolongum, Bifidobacterium pseudocatenulatum, Bifidobacterium lactis, Bifidobacterium animalis, Bifidobacterium magnum* and *Bifidobacterium mongoliense.*

Examples of the *Leuconostoc* bacteria include *Leuconostoc carnosum, Leuconostoc citreum, Leuconostoc lactis, Leuconostoc mesenteroides* subsp. *Cremoris* and *Leuconostoc pseudomesenteroides.*

Examples of the *Pediococcus* bacteria include *Pediococcus acidilactici, Pediococcus argentinicus, Pediococcus damnosus, Pediococcus pentosaceus* and *Pediococcus stilesii.*

Examples of the *Weissella* bacteria include *Weissella cibaria, Weissella confusa,* and *Weissella paramesenteroides.*

### [1.2. Protease]

The protease to be used in a method according to an embodiment of the present invention is not particularly limited. Examples of the protease include serine protease, cysteine protease, threonine protease, asparagine protease, aspartic protease, glutamic protease and metalloprotease. Of these, one or more selected from the group consisting of serine protease, aspartic protease and metalloprotease are preferable.

The protease may be derived from any organism. Examples of the organism from which protease is derived include higher organisms, bacteria and fungi. In an embodiment, the protease is derived from *Bacillus licheniformis.* In an embodiment, the protease is derived from *Bacillus subtilis.* In an embodiment, protease is derived from *Aspergillus oryzae.*

Examples of the protease include trypsin, chymotrypsin, elastase, papain, bromelain, calpain, lysosomal cathepsins, pepsin, rennin, thermolysin, carboxypeptidase A, collagenase and plasmin. A commercially available protease preparation may be used. Examples of the commercially available protease preparation include protease M "Amano" SD, PROTIN SD-AY10, THERMOASE PC10F, PROTIN SD-NY10 and PROTACs (these are manufactured by Amano Enzyme Inc); SUMIZYME LPL-G, SUMIZYME MP, SUMIZYME BNP, SUMIZYME FP-G and SUMIZYME LP-G (these are manufactured by SHINNIHON CHEMICALS Corporation). A protease commonly known is available as a commercially available protease preparation. Examples of the preparation include pepsin, papain and bromelain (these are manufactured by FUJIFILM Wako Pure Chemical Corporation).

Examples of preferable proteases among those mentioned above include PROTIN SD-AY10 (manufactured by Amano Enzyme Inc.; serine protease derived from *Bacillus licheniformis*)*,* SUMIZYME BNP (manufactured by SHINNIHON CHEMICALS Corporation; metalloprotease derived from *Bacillus subtilis*), SUMIZYME LP-G (manufactured by SHINNIHON CHEMICALS Corporation; a mixture of serine protease, aspartic protease and metalloprotease derived from *Aspergillus oryzae*)*.* The proteins derived from microorganisms (in particular, beer yeast) hydrolyzed by these enzymes have a high content of a dipeptide and a high DPP-4 inhibitory activity.

Examples of other proteases that can be used include Protease M "Amano" SD (manufactured by Amano Enzyme Inc.; a mixture of aspartic protease and serine protease derived from *Aspergillus oryzae*), PROTACs (manufactured by Amano Enzyme Inc.; a mixture of aspartic protease and serine protease derived from *Aspergillus oryzae*), SUMIZYME LPL-G (manufactured by SHINNIHON CHEMICALS Corporation; aspartic protease derived from *Aspergillus oryzae*), SUMIZYME FP-G (manufactured by SHINNIHON CHEMICALS Corporation; a mixture of serine protease, aspartic protease and metalloprotease derived from *Aspergillus oryzae*), THERMOASE PC10F (manufactured by Amano Enzyme Inc.; serine protease derived from *GeoBacillus stearothermophilus*), PROTIN SD-NY10 (manufactured by Amano Enzyme Inc.; metalloprotease derived from *Bacillus amyloliquefaciens*), SUMIZYME MP (manufactured by SHINNIHON CHEMICALS Corporation; serine protease derived from *Aspergillus melleus*), pepsin, papain and bromelain. In an embodiment, pepsin is aspartic protease derived from the swine gastric mucosa. In an embodiment, papain is cysteine protease derived from a papaya fruit. In an embodiment, bromelain is cysteine protease derived from a pineapple fruit or leaves thereof.

### [1.3. Hydrolysis condition]

The condition for hydrolyzing a protein derived from a microorganism is not particularly limited. For example, temperature and pH may be set so as to fit optimal conditions for the protease to be used. In the method according to an embodiment of the present invention, a DPP-4 inhibitor can be produced by any one of the enzymes having optimum pH in an acidic range, a neutral range and a basic range.

The amount of the protease to be used can be appropriately set depending on the type of protease. For example, the lower limit of the protease to be added is preferably 0.01 part by weight or more, more preferably 0.1 part by weight or more, and further preferably 1 part by weight or more, based on 100 parts by weight of the amount of the protein derived from a microorganism. The upper limit of the protease to be added is preferably 10 parts by weight or less, and more preferably, 5 parts by weight or less, based on 100 parts by weight of the amount of the protein derived from a microorganism.

The time required for hydrolysis can be appropriately set depending on the amount of protein derived from a microorganism and the type of protease. For example, the lower limit of the hydrolysis time is preferably one hour or more, more preferably 4 hours or more, and further preferably 16 hours or more. The upper limit of the hydrolysis time can be, for example, one week or less.

A production method according to an embodiment of the present invention may include steps other than the step mentioned above. Examples of the other steps include culturing a microorganism; separating the microorganism; extracting a protein derived from the microorganism; pretreating the protein derived from a microorganism (for example, a treatment with an acid or a base); and purifying, concentrating, fractioning, drying and grinding a resulting hydrolysate.

### [2. DPP-4 inhibitor]

### [2.1. Components of DPP-4 inhibitor]

The DPP-4 inhibitor according to an aspect of the present invention contains a hydrolysate obtained by hydrolyzing a protein derived from a microorganism with a protease. Since the hydrolysate contains a wide variety of peptide components, it is not realistic to identify which one of the peptide components (or which combination of the components) exhibits a DPP-4 inhibitory activity. The present inventors presume that a dipeptide contained in the hydrolysate highly possibly has a DPP-4 inhibitory activity but the mechanism of action presumed should not be construed as limiting an aspect of the present invention. More specifically, not a dipeptide but an oligopeptide may have a DPP-4 inhibitory activity or a completely different mechanism of action may present.

The total amount of dipeptides contained in the supernatant of a hydrolysate is preferably 1 µg or more, more preferably 10 µg or more and further preferably 100 µg or more per mL.

In an embodiment, a hydrolysate contains one type or more, 2 types or more, 3 types or more, 4 types or more, 5 types or more, 6 types or more, 7 types or more, 8 types or more, 9 types or more or all types of dipeptides among the following dipeptides. The total amount of these dipeptides is preferably 1 µg or more, more preferably 10 µg or more, and further preferably 100 µg or more per supernatant (mL) of the hydrolysate. Note that, in the specification, the amino acid sequence of a dipeptide, etc., is represented by one letter codes of amino acids.

Dipeptides: VW, EF, VY, TY, TF, VN, AA, SL, GF and VL.

In an embodiment, a hydrolysate contains one type or more, 2 types or more, 3 types or more, 4 types or more, 5 types or more, 6 types or more, 7 types or more, 8 types or more, 9 types or more or all types of dipeptides among the following dipeptides. The total amount of these dipeptides is preferably 1 µg or more, more preferably 10 µg or more, and further preferably 100 µg or more per supernatant (mL) of the hydrolysate.

Dipeptides: VW, VY, TY, VN, AN, LN, AA, LA, EF and IA.

In an embodiment, a hydrolysate contains one type or more, 2 types or more, 3 types or more, 4 types or more, 5 types or more, 6 types or more, 7 types or more, 8 types or more, 9 types or more or all types of dipeptides among the following dipeptides. The total amount of these dipeptides is preferably 1 µg or more, more preferably 10 µg or more, and further preferably 100 µg or more per supernatant (mL) of the hydrolysate.

Dipeptides: DP, AP, EF, KN, HP, DA, VP, AA, GN and LE.

In an embodiment, a hydrolysate contains one type or more, 2 types or more, 3 types or more, 4 types or more, 5 types or more, 6 types or more, 7 types or more, 8 types or more, 9 types or more or all types of dipeptides among the following dipeptides. The total amount of these dipeptides is preferably 1 µg or more, more preferably 10 µg or more, and further preferably 100 µg or more per supernatant (mL) of the hydrolysate.

Dipeptide: DP, EF, AP, KN, DA, AA, VN, VP, DL and SN.

In an embodiment, a hydrolysate contains one type or more, 2 types or more, 3 types or more, 4 types or more, 5 types or more, 6 types or more, 7 types or more, 8 types or more, 9 types or more or all types of dipeptides among the following dipeptides. The total amount of these dipeptides is preferably 1 µg or more, more preferably 10 µg or more, and further preferably 100 µg or more per supernatant (mL) of the hydrolysate.

Dipeptides: VW, VY, EF, LE, IA, TY, TF, DP, FG and SY.

In an embodiment, a hydrolysate contains one type or more, 2 types or more, 3 types or more, 4 types or more, 5 types or more, 6 types or more, 7 types or more, 8 types or more, 9 types or more or all types of dipeptides among the following dipeptides. The total amount of these dipeptides is preferably 1 µg or more, more preferably 10 µg or more, and further preferably 100 µg or more per supernatant (mL) of the hydrolysate.

Dipeptides: GY, EF, VW, VY, LE, IA, TY, TF, DP and FG.

In an embodiment, a hydrolysate contains one type or more, 2 types or more, 3 types or more, 4 types or more, 5 types or more, 6 types or more, 7 types or more, 8 types or more, 9 types or more or all types of dipeptides among the following dipeptides. The total amount of these dipeptides is preferably 1 µg or more, more preferably 10 µg or more, and further preferably 100 µg or more per supernatant (mL) of the hydrolysate.

Dipeptides: DP, AP, EF, DA, HP, AA, VP, GN, VE and AN.

In an embodiment, a hydrolysate contains one type or more, 2 types or more, 3 types or more, 4 types or more, 5 types or more, 6 types or more, 7 types or more, 8 types or more, 9 types or more or all types of dipeptides among the following dipeptides. The total amount of these dipeptides is preferably 1 µg or more, more preferably 10 µg or more, and further preferably 100 µg or more per supernatant (mL) of the hydrolysate.

Dipeptides: VW, VY, FG, LA, LE, AN, VN, FN, LN and AR.

In an embodiment, a hydrolysate contains one type or more, 2 types or more, 3 types or more, 4 types or more, 5 types or more, 6 types or more, 7 types or more, 8 types or more, 9 types or more or all types of dipeptides among the following dipeptides. The total amount of these dipeptides is preferably 1 µg or more, more preferably 10 µg or more, and further preferably 100 µg or more per supernatant (mL) of the hydrolysate.

Dipeptides: VW, VY, TY, VN, LN, EF, AN, NA, IA and LA.

In an embodiment, a hydrolysate contains one type or more, 2 types or more, 3 types or more, 4 types or more, 5 types or more, 6 types or more, 7 types or more, 8 types or more, 9 types or more or all types of dipeptides among the following dipeptides. The total amount of these dipeptides is preferably 1 µg or more, more preferably 10 µg or more, and further preferably 100 µg or more per supernatant (mL) of the hydrolysate.

Dipeptides: DP, AP, KN, NA, DA, HP, EF, GN, VP and WG.

In an embodiment, a hydrolysate contains one type or more, 2 types or more, 3 types or more, 4 types or more, 5 types or more, 6 types or more, 7 types or more, 8 types or more, 9 types or more or all types of dipeptides among the following dipeptides. The total amount of these dipeptides is preferably 1 µg or more, more preferably 10 µg or more, and further preferably 100 µg or more per supernatant (mL) of the hydrolysate.

Dipeptides: VW, VN, TY, NA, AA, LE, AS, AR, FG and LA.

In an embodiment, a hydrolysate contains one type or more, 2 types or more, 3 types or more, 4 types or more, 5 types or more, 6 types or more, 7 types or more, 8 types or more, 9 types or more or all types of dipeptides among the following dipeptides. The total amount of these dipeptides is preferably 1 µg or more, more preferably 10 µg or more, and further preferably 100 µg or more per supernatant (mL) of the hydrolysate.

Dipeptides: VW, LN, RA, RP, VN, AP, LE, LS, AS and FG.

The types and amounts of dipeptides contained in a hydrolysate can be measured by a known method (see, for example, Examples of the specification of the present application).

### [2.2. Effect of DPP-4 inhibitor]

A DPP-4 inhibitor inhibits one or more of the functions that DPP-4 has. In an embodiment, a DPP-4 inhibitor inhibits the cleavage of a peptide bond between a proline or alanine residue at the second position from the N terminal of a peptide chain and an amino acid residue at the third position from the N terminal.

In an embodiment, the inhibitory activity of a DPP-4 inhibitor is represented by the difference in the function of DPP-4 between a system containing a DPP-4 inhibitor and a system not containing the DPP-4 inhibitor. The system containing a DPP-4 inhibitor preferably inhibits 40% or more, more preferably 50% or more, further preferably 60% or more, and particularly preferably 70% or more of the function of DPP-4, in comparison with the system not containing the DPP-4 inhibitor. The inhibitory activity of DPP-4 is measured by a known kit (see, for example, Examples).

### [2.3. Application of DPP-4 inhibitor]

An aspect of the present invention is directed to an article containing a DPP-4 inhibitor mentioned above. Examples of the article include pharmaceuticals, food supplements, beverages, foods, veterinary medicines, animal supplements, animal drinks and feeds. These articles can be used for preventing and/or improving abnormality involving DPP-4. Since methods for producing articles containing a DPP-4 inhibitor are known to those skilled in the art, detailed descriptions thereof are omitted.

An aspect of the present invention is directed to a method for preventing and/or improving abnormality involving DPP-4, including a step of applying an effective amount of the DPP-4 inhibitor mentioned above to a subject. The DPP-4 inhibitor to be applied may take the form of a pharmaceutical, a food supplement, a beverage, a food, a veterinary medicine, an animal supplement, an animal drink or food. In an embodiment, the subject is a human. In an embodiment, the subject is an animal except a human.

In the specification, "for animals" is intended to mean "for animals except humans". Examples of the animals except for humans include animals of the Artiodactyla (e.g., cow, boar, pig, sheep, goat), animals of the Perissodactyla (e.g., horse), rodents (e.g., mouse, rat, hamster, squirrel), animals of the Lagomorpha (e.g., rabbit) and predatory animals (e.g., dog, cat, ferret). Not only livestock or companion animals (pet animals) but also wild animals are included in the animals except humans.

Examples of the abnormality involving DPP-4 include hyperglycemia and diabetes (e.g., type 1 diabetes, type 2 diabetes). In an embodiment, the abnormality involving DPP-4 is hyperglycemia. In an embodiment, the abnormality involving DPP-4 is diabetes (for example, type 2 diabetes).

The contents described in the above sections may appropriately apply to other sections. An aspect of the present invention is not limited to individual embodiments mentioned above and may be modified in various ways within the range of the claims. Accordingly, embodiments obtained by appropriately combining technical means disclosed in different embodiments are also included in the technical scope according to the aspect of the present invention.

All scientific literatures and patent literatures described in the specification are incorporated herein by reference.

### [Summary]

An aspect of the present invention includes the followings.
<1> A dipeptidyl peptidase IV (DPP-4) inhibitor comprising a hydrolysate obtained by hydrolyzing a protein derived from a microorganism with a protease.
<2> The DPP-4 inhibitor according to <1>, wherein the microorganism is one or more selected from the group consisting of a yeast and a lactic acid bacterium.
<3> The DPP-4 inhibitor according to <2>, wherein
   the yeast is one or more selected from the group consisting of beer yeast, baker's yeast and torula yeast, and
   the lactic acid bacterium is one or more selected from the group consisting of lactic acid bacteria belonging to the genus *Lactobacillus,* the genus *Lactococcus* and the genus *Enterococcus.*
<4> The DPP-4 inhibitor according to any one of <1> to <3>, wherein the protease is one or more selected from the group consisting of serine protease, aspartic protease, metalloprotease and cysteine protease.
<5> The DPP-4 inhibitor according to any one of <1> to <4>, wherein the protease is a protease derived from *Bacillus licheniformis* or *Bacillus subtilis.*
<6> The DPP-4 inhibitor according to <5>, wherein the protease is one or more selected from the group consisting of serine protease derived from *Bacillus licheniformis* and metalloprotease derived from *Bacillus subtilis.*
<7> The DPP-4 inhibitor according to any one of <1> to <4>, wherein the protease is a protease derived from *Aspergillus oryzae.*
<8> The DPP-4 inhibitor according to <7>, wherein the protease is a mixture of serine protease, aspartic protease and metalloprotease derived from *Aspergillus oryzae.*
<9> The DPP-4 inhibitor according to any one of <1> to <4>, wherein the protease is one or more selected from the group consisting of the following (a) to (h):
   (a) aspartic protease derived from *Aspergillus oryzae;*
   (b) a mixture of aspartic protease and serine protease derived from *Aspergillus oryzae;*
   (c) serine protease derived from *GeoBacillus stearothermophilus;*
   (d) metalloprotease derived from *Bacillus amyloliquefaciens;*
   (e) serine protease derived from *Aspergillus melleus;*
   (f) pepsin;
   (g) papain; and
   (h) bromelain.
<10> An article for preventing and/or improving abnormality involving DPP-4, comprising a DPP-4 inhibitor according to any one of <1> to <9>.
<11> The article according to <10>, wherein the abnormality involving DPP-4 is hyperglycemia.
<12> The article according to <11>, wherein the hyperglycemia is hyperglycemia due to type 2 diabetes.
<13> A method for producing a dipeptidyl peptidase IV (DPP-4) inhibitor, comprising a step of hydrolyzing a protein derived from a microorganism with a protease.
<14> The production method according to <13>, wherein the microorganism is one or more selected from the group consisting of a yeast and a lactic acid bacterium.
<15> The production method according to <14>, wherein
   the yeast is one or more selected from the group consisting of beer yeast, baker's yeast and torula yeast, and
   the lactic acid bacterium is one or more selected from the group consisting of lactic acid bacteria belonging to the genus *Lactobacillus,* the genus *Lactococcus* and the genus *Enterococcus.*
<16> The production method according to any one of <13> to <15>, wherein the protease is one or more selected from the group consisting of serine protease, aspartic protease, metalloprotease and cysteine protease.
<17> The production method according to any one of <13> to <16>, wherein the protease is a protease derived from *Bacillus licheniformis* or *Bacillus subtilis.*
<18> The production method according to <17>, wherein the protease is one or more selected from the group consisting of serine protease derived from *Bacillus licheniformis* and metalloprotease derived from *Bacillus subtilis.*
<19> The production method according to any one of <13> to <16>, wherein the protease is a protease derived from *Aspergillus oryzae.*
<20> The production method according to <19>, wherein the protease is a mixture of serine protease, aspartic protease and metalloprotease derived from *Aspergillus oryzae.*
<21> The production method according to any one of <13> to <16>, wherein the protease is one or more selected from the group consisting of the following (a) to (h):
   (a) aspartic protease derived from *Aspergillus oryzae*;
   (b) a mixture of aspartic protease and serine protease derived from *Aspergillus oryzae;*
   (c) serine protease derived from *GeoBacillus stearothermophilus;*
   (d) metalloprotease derived from *Bacillus amyloliquefaciens;*
   (e) serine protease derived from *Aspergillus melleus;*
   (f) pepsin;
   (g) papain; and
   (h) bromelain.

An aspect of the present invention includes the followings.
<1A> A method for preventing and/or improving abnormality involving DPP-4, comprising a step of applying a hydrolysate obtained by hydrolyzing a protein derived from a microorganism with a protease to a subject.
<2A> A method for preventing or improving abnormality involving DPP-4, comprising a step of applying the DPP-4 inhibitor according to any one of <1> to <7> to a subject.
<3A> The method according to <1A> or <2A>, wherein the abnormality involving DPP-4 is hyperglycemia.
<4A> The method according to <3A>, wherein the hyperglycemia is hyperglycemia due to type 2 diabetes.
<1B> A DPP-4 inhibitor comprising a hydrolysate obtained by hydrolyzing a protein derived from a microorganism with a protease, for use in preventing and/or improving abnormality involving DPP-4.
<2B> The DPP-4 inhibitor according to any one of <1> to <7>, for use in preventing and/or improving abnormality involving DPP-4.
<3B> The DPP-4 inhibitor according to <1B> or <2B>, wherein the abnormality involving DPP-4 is hyperglycemia.
<4B> The DPP-4 inhibitor according to <3B>, wherein the hyperglycemia is hyperglycemia due to type 2 diabetes.
<1C> Use of a DPP-4 inhibitor comprising a hydrolysate obtained by hydrolyzing a protein derived from a microorganism with a protease, for producing an agent for preventing and/or improving abnormality involving DPP-4.
<2C> The use of a DPP-4 inhibitor according to any one of <1> to <7>, for producing an agent for preventing and/or improving abnormality involving DPP-4.
<3C> The use according to <1C> or <2C>, wherein the abnormality involving DPP-4 is hyperglycemia.
<4C> The use according to <3C>, wherein the hyperglycemia is hyperglycemia due to type 2 diabetes.

### Examples

Now, an aspect of the present invention will be more specifically described by way of Examples but the aspect of the present invention is not limited to the following Examples, alone.

### [Materials]

### [1. Microorganisms]

The following yeasts and lactic acid bacteria were used.
- Beer yeast (protein content: about 50%): dry yeast Ebios specified in the Japanese Pharmacopoeia.
- Baker's yeast TP (protein content: about 50%): available from MIWA SEIYAKU CO., LTD.
- Torula yeast (protein content: about 50%): available from MIWA SEIYAKU CO., LTD.
- Lactic acid bacteria: *Lactobacillus plantarum, Lactococcus lactis, Enterococcus faecalis* and *Lactobacillus sakei.* These were prepared in accordance with the following procedure.

The procedure for preparing microbial cells of lactic acid bacteria is as follows. In the following procedure, centrifugation conditions were all 6,000 × g for 30 minutes.

### (Preparation of Lactobacillus plantarum)

(1) *Lactobacillus plantarum* was put in MRS medium and cultured at 30°C for 24 hours. OD600 after completion of culture was 12.4.
(2) 3 L of the culture solution was centrifuged.
(3) The precipitate obtained was suspended by adding 400 mL of water. Thereafter, the suspension was centrifuged and washed. Step 3 was repeated twice in total.
(4) The precipitate obtained was suspended by adding 400 mL of water. The suspension was sterilized in an autoclave.
(5) After completion of sterilization, the suspension was centrifuged and the precipitate obtained was lyophilized.

### (Preparation of Lactococcus lactis and Enterococcus faecalis)

*Lactococcus lactis* or *Enterococcus faecalis* were cultured in BHI medium. The culture solution (1 L) obtained was treated in the same manners as in the above steps (2) to (5).

### (Preparation of Lactobacillus sakei)

*Lactobacillus sakei* was cultured in MRS medium. The culture solution (1 L) obtained was treated in the same manners as in the above steps (2) to (5).

### [2. Protease]

The enzymes listed in the following Table 1 were used.

### [Table 1]

**Table 1**

| No. | Name | Manufacturer | Derivation | Reaction pH | Specific activity |
|---|---|---|---|---|---|
| A1 | Protease M "Amano" SD | Amano Enzyme Inc | *Aspergillus oryzae* | 4.0 | 40, 000 |
| A2 | SUMIZYME LPL-G | SHINNIHON CHEMICALS Corporation | *Aspergillus oryzae* | 4.0 | 500, 000 |
| A3 | Pepsin | FUJIFILM Wako Pure Chemical Corporation | Swine gastric mucosa | 4.0 | 10, 000 |
| B1 | PROTIN SD-AY10 | Amano Enzyme Inc | *Bacillus licheniformis* | 9.0 | 80, 000 |
| B2 | SUMIZYME MP | SHINNIHON CHEMICALS Corporation | *Aspergillus melleus* | 9.0 | 130, 000 |
| N1 | THERMOASE PC10F | Amano Enzyme Inc | *Geobacillus stearothermophilus* | 7.0 | 90, 000 |
| N2 | PROTIN SD-NY10 | Amano Enzyme Inc | *Bacillus amyloliquefaciens* | 7.0 | 70, 000 |
| N3 | PROTACs | Amano Enzyme Inc | *Aspergillus oryzae* | 7.0 | 1, 400 |
| N4 | SUMIZYME BNP | SHINNIHON CHEMICALS Corporation | *Bacillus subtilis* | 7.0 | 70, 000 |
| N5 | SUMIZYME FP-G | SHINNIHON CHEMICALS Corporation | *Aspergillus oryzae* | 7.0 | 50, 000 |
| N6 | SUMIZYME LP-G | SHINNIHON CHEMICALS Corporation | *Aspergillus oryzae* | 7.0 | 75, 000 |
| N7 | Papain | FUJIFILM Wako Pure Chemical Corporation | Papaya fruit | 7 0 | |
| N8 | Bromelain | FUJIFILM Wako Pure Chemical Corporation | Pineapple fruit and leaves | 7.0 | |

### [3. Liquid pH-adjuster]

A liquid pH-adjuster was prepared by mixing 6 N HCl and 6 N NaOH. Three types of liquid pH-adjusters different in pH: 4.0, 7.0 and 9.0, were prepared. A liquid pH-adjuster may be prepared by diluting a diluted HCl aqueous solution or a diluted NaOH aqueous solution with water.

### [Method]

### [1. Treatment of microorganism with protease]

(1) To 20 mg of a microbial sample, 1 mL of a liquid pH-adjuster was added. As the liquid pH-adjuster to be added, a liquid pH adjuster having pH at which a protease undergoes a reaction, was used. The 20 mg/mL suspension of the microorganism thus obtained was designated as solution A.
(2) To 10 mg of a protease, 1 mL of a liquid pH-adjuster was added. As the liquid pH-adjuster to be added, a liquid pH adjuster having pH at which the protease for use in treatment undergoes a reaction, was used. The 10 mg/mL solution of the protease thus obtained was designated as solution B.
(3) To the whole amount of solution A, 10 µL of solution B was added. In the case of, e.g., beer yeast, since the protein content is about 50%, the weight ratio of an enzyme to the protein is about 1%. The protein content of a lactic acid bacterium was not measured but assumed to be the same (about 50%) as in yeast.
(4) The microorganism was subjected to a hydrolysis treatment at 55°C for 16 hours.
(5) A heat treatment was applied at 95°C for 15 minutes to inactivate an enzyme. Thereafter, the sample was refrigerated.
(6) The sample was centrifuged (15,000 × g, 5 minutes). The supernatant (10 µL) was taken and used for measurement of DPP-4 inhibitory activity.

### [2. Measurement of DPP-4 inhibitory activity]

Dipeptidyl peptidase IV (DPP4) Inhibitor Screening Assay Kit (ab133081) manufactured by abcam was used as a measurement kit. The kit contains the following materials.
- DPP Assay Buffer
- DPP4 (human recombinant)
- DPP Substrate
- Sitagliptin Positive Control Inhibitor

As a plate reader, SpectraMax i3 (manufactured by Molecular Devices) was used.

The details of a procedure for measuring inhibitory activity are as follows.
(1) In a 96-well plate, sample wells, a background well and 100% initial-activity well were provided. The following materials were poured in respective wells.
   · Sample well: 30 µL of DPP Assay Buffer, 10 µL of DPP4 (human recombinant), and 10 µL of a sample
   · Background well: 40 µL of DPP Assay Buffer, and 10 µL of solvent (the same solvent used for diluting a sample)
   · 100% initial-activity well: 30 µL of DPP Assay Buffer, 10 µL of DPP4 (human recombinant), and 10 µL of solvent (the same solvent used for diluting a sample)
(2) To each of the wells, 50 µL of DPP Substrate was added to initiate a reaction. The plate was covered and subjected to incubation performed at 37°C for 30 minutes.
(3) The fluorescence intensity of each of the wells was measured by a plate reader. The wavelength of excitation light was 355 nm and the wavelength of synchrotron radiation was 460 nm.

### [Example 1]

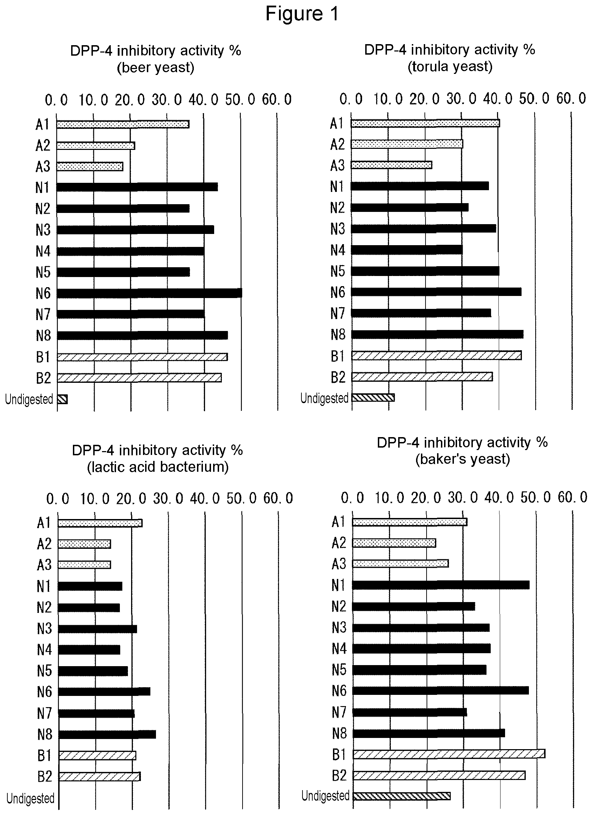

Beer yeast, baker's yeast, torula yeast and a lactic acid bacterium (*Lactobacillus plantarum*) were subjected to a hydrolysis treatment with 13 types of proteases listed in Table 1. The DPP-4 inhibitory activities of the hydrolysates obtained are shown in Figure 1. The DPP-4 inhibitory activities of the beer-yeast hydrolysates are more specifically shown in Table 2. The DPP-4 inhibitory activity of undigested beer yeast was measured and used as a control in the experiment.

### [Table 2]

**Table 2**

| No. | Name | DPP-4 inhibitory activity (%) |
|---|---|---|
| A1 | Protease M "Amano" SD | 65.9 |
| A2 | SUMIZYME LPL-G | 59.7 |
| A3 | Pepsin | 43.0 |
| B1 | PROTIN SD-AY10 | 75.3 |
| B2 | SUMIZYME MP | 69.2 |
| N1 | THERMOASE PC10F | 66.3 |
| N2 | PROTIN SD-NY10 | 68.0 |
| N3 | PROTACs | 83.5 |
| N4 | SUMIZYME BNP | 70.0 |
| N5 | SUMIZYME FP-G | 58.7 |
| N6 | SUMIZYME LP-G | 75.6 |
| N7 | Papain | 74.5 |
| N8 | Bromelain | 79.2 |
| | Undigested beer yeast | 2.2 |

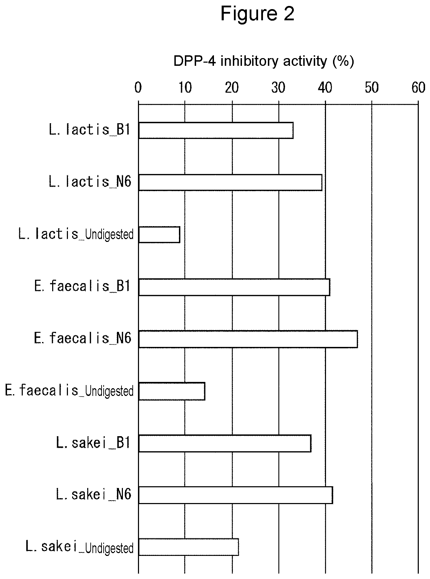

Lactic acid bacteria (*Lactococcus lactis, Enterococcus faecalis* and *Lactobacillus sakei*) were subjected to a hydrolysis treatment with each of PROTINSD-AY10 and SUMIZYME LP-G (corresponding to B1 and N6 shown in Table 1, respectively). The DPP-4 inhibitory activities of the hydrolysates obtained are shown in Table 3 and Figure 2. The DPP-4 inhibitory activities of undigested lactic acid bacteria were measured and used as a control in the experiment.

### [Table 3]

**Table 3**

| Lactic acid bacteria | Protease | DPP-4 inhibitory activity |
|---|---|---|
| *Lactococcus lactis* | PROTIN SD-AY10 | 33.2 |
| *Lactococcus lactis* | SUMIZYME LP-G | 39.4 |
| *Lactococcus lactis* | Undigested | 8.8 |
| *Enterococcus faecalis* | PROTIN SD-AY10 | 41.0 |
| *Enterococcus faecalis* | SUMIZYME LP-G | 46.9 |
| *Enterococcus faecalis* | Undigested | 14.2 |
| *Lactobacillus sakei* | PROTIN SD-AY10 | 37.0 |
| *Lactobacillus sakei* | SUMIZYME LP-G | 41.6 |
| *Lactobacillus sakei* | Undigested | 21.4 |

### [Results]

From Figures 1 and 2 and Tables 2 and 3, it was found that DPP-4 inhibitors can be produced by subjecting cells of various microorganisms to hydrolysis with various proteases. From Figures 1 and 2, it was found that the hydrolysates obtained by subjecting a yeast to hydrolysis with a protease tend to have high DPP-4 inhibitory activities.

### [Example 2]

Beer yeast was subjected to a hydrolysis treatment with 12 types of proteases of those shown in Table 1.

The types and amounts of dipeptides contained in the hydrolysates obtained were analyzed. The details of the procedure are as follows.
(1) A sample was centrifuged at 15000 × g for 5 minutes.
(2) The supernatant was pre-treated and analyzed by the LC/MS/MS method.

Top 10 dipeptides of each of the hydrolysates are listed in descending order in Table 4. Of these, the numbers of the dipeptides classified into "highly active dipeptide" in Reference Example (later described) were listed in Table 4.

In addition, the total amount (µg/ mL) of the dipeptides contained in each of the hydrolysates is shown in Table 4.

### [Table 4]

**Table 4**

| Protease | | A1 | A2 | A3 | B1 | B2 | N1 | N2 | N4 | N5 | N6 | N7 | N8 |
|---|---|---|---|---|---|---|---|---|---|---|---|---|---|
| Ranking | 1 | DP | VW | GY | VW | DP | VW | VW | VW | DP | DP | VW | VW |
| | 2 | EF | VY | EF | EF | AP | VY | VY | VY | AP | AP | VN | LN |
| | 3 | AP | EF | VW | VY | EF | FG | TY | TY | KN | EF | TY | RA |
| | 4 | KN | LE | VY | TY | DA | LA | VN | VN | NA | KN | NA | RP |
| | 5 | DA | IA | LE | TF | HP | LE | LN | AN | DA | HP | AA | VN |
| | 6 | AA | TY | IA | VN | AA | AN | EF | LN | HP | DA | LE | AP |
| | 7 | VN | TF | TY | AA | VP | VN | AN | AA | EF | VP | AS | LE |
| | 8 | VP | DP | TF | SL | GN | FN | NA | LA | GN | AA | AR | LS |
| | 9 | DL | FG | DP | GF | VE | LN | IA | EF | VP | GN | FG | AS |
| | 10 | SN | SY | FG | VL | AN | AR | LA | IA | WG | LE | LA | FG |
| DPP-4 inhibitory activity | | 66 | 60 | 43 | 75 | 69 | 66 | 68 | 70 | 59 | 76 | 75 | 79 |
| Total amount of dipeptides (µg/mL) | | 1489 | 617 | 2 | 2801 | 1055 | 570 | 1023 | 1581 | 2064 | 2164 | 548 | 173 |
| Number of highly active dipeptides | | 1 | 1 | 1 | 0 | 1 | 1 | 2 | 2 | 1 | 1 | 1 | 1 |

### [Reference Example]

The DPP-4 inhibitory activities of commercially available synthetic dipeptides were measured. More specifically, 200 µg/ mL solutions of dipeptides were used as samples and DPP-4 inhibitory activities thereof were measured by the method mentioned above. Dipeptides having a relatively high DPP-4 inhibitory activity (70% or more) are shown in Table 5.

### [Table 5]

**Table 5**

| Dipeptide | KP | IP | VP | WP | WA | VA | WR | RL | WV | WK | WL | LL | IA |
|---|---|---|---|---|---|---|---|---|---|---|---|---|---|
| DPP-4 inhibitory activity | 105.3 | 103.7 | 98.7 | 96.6 | 96.5 | 95.8 | 95.0 | 93.9 | 90.0 | 89.5 | 88.7 | 87.5 | 87.2 |
| Dipeptide | MS | ML | YM | WI | MA | MP | HW | WH | MI | LA | YP | FP | YA |
| DPP-4 inhibitory activity | 86.8 | 86. 7 | 86.0 | 85.5 | 84.5 | 84.2 | 79.3 | 78.4 | 77.8 | 76.1 | 75.4 | 73.9 | 73.8 |
| Dipeptide | MK | LP | WN | RP | MW | IV | MM | MV | LW | WM | | | |
| DPP-4 inhibitory activity | 73.7 | 73.4 | 73.4 | 73.1 | 72.5 | 72.5 | 71.9 | 71.7 | 71.2 | 70.6 | | | |

### [Results]

From Table 4, it was found that hydrolysates of B1 (PROTINSD-AY10), N4 (SUMIZYME BNP) and N6 (SUMIZYME LP-G) among the proteases examined in Example 2, have high DPP-4 inhibitory activities and the total amounts of the dipeptides contained therein are high. From this, it is suggested that when a beer-yeast hydrolysate is used as a DPP-4 inhibitor, PROTINSD-AY10 (serine protease derived from *Bacillus licheniformis*), SUMIZYME BNP (metalloprotease derived from *Bacillus subtilis*) and SUMIZYME LP-G (a mixture of serine protease, aspartic protease and metalloprotease derived from *Aspergillus oryzae*) are preferably employed as the protease.

Similarly, from Table 4, it is apparent that a dipeptide having a high DPP-4 inhibitory activity was not contained in the dipeptide profile of the hydrolysates of beer yeast with B1 (PROTINSD-AY10). Despite that, a high DPP-4 inhibitory activity was shown. This was a surprising result that cannot be expected by those skilled in the art.

### Industrial Applicability

An aspect of the present invention is expected to be used for prevention and/or improvement of, for example, hyperglycemia.

## Claims

1. A dipeptidyl peptidase IV (DPP-4) inhibitor comprising a hydrolysate obtained by hydrolyzing a protein derived from a microorganism with a protease.

2. The DPP-4 inhibitor according to claim 1, wherein the microorganism is one or more selected from the group consisting of a yeast and a lactic acid bacterium.

3. The DPP-4 inhibitor according to claim 2, wherein
the yeast is one or more selected from the group consisting of beer yeast, baker's yeast and torula yeast, and
the lactic acid bacterium is one or more selected from the group consisting of lactic acid bacteria belonging to the genus *Lactobacillus,* the genus *Lactococcus* and the genus *Enterococcus.*

4. The DPP-4 inhibitor according to any one of claims 1 to 3, wherein the protease is one or more selected from the group consisting of serine protease, aspartic protease, metalloprotease and cysteine protease.

5. The DPP-4 inhibitor according to any one of claims 1 to 4, wherein the protease is a protease derived from *Bacillus licheniformis* or *Bacillus subtilis.*

6. The DPP-4 inhibitor according to claim 5, wherein the protease is one or more selected from the group consisting of serine protease derived from *Bacillus licheniformis* and metalloprotease derived from *Bacillus subtilis.*

7. The DPP-4 inhibitor according to any one of claims 1 to 4, wherein the protease is a protease derived from *Aspergillus oryzae.*

8. The DPP-4 inhibitor according to claim 7, wherein the protease is a mixture of serine protease, aspartic protease and metalloprotease derived from *Aspergillus oryzae.*

9. The DPP-4 inhibitor according to any one of claims 1 to 4, wherein the protease is one or more selected from the group consisting of the following (a) to (h):
(a) aspartic protease derived from *Aspergillus oryzae;*
(b) a mixture of aspartic protease and serine protease derived from *Aspergillus oryzae;*
(c) serine protease derived from *GeoBacillus stearothermophilus;*
(d) metalloprotease derived from *Bacillus amyloliquefaciens;*
(e) serine protease derived from *Aspergillus melleus;*
(f) pepsin;
(g) papain; and
(h) bromelain.

10. An article for preventing and/or improving abnormality involving DPP-4, comprising a DPP-4 inhibitor according to any one of claims 1 to 9.

11. The article according to claim 10, wherein the abnormality involving DPP-4 is hyperglycemia.

12. The article according to claim 11, wherein the hyperglycemia is hyperglycemia due to type 2 diabetes.

13. A method for producing a dipeptidyl peptidase IV (DPP-4) inhibitor, comprising a step of hydrolyzing a protein derived from a microorganism with a protease.

14. The production method according to claim 13, wherein the microorganism is one or more selected from the group consisting of a yeast and a lactic acid bacterium.

15. The production method according to claim 14, wherein
the yeast is one or more selected from the group consisting of beer yeast, baker's yeast and torula yeast, and
the lactic acid bacterium is one or more selected from the group consisting of lactic acid bacteria belonging to the genus *Lactobacillus,* the genus *Lactococcus* and the genus *Enterococcus.*

16. The production method according to any one of claims 13 to 15, wherein the protease is one or more selected from the group consisting of serine protease, aspartic protease, metalloprotease and cysteine protease.

17. The production method according to any one of claims 13 to 16, wherein the protease is a protease derived from *Bacillus licheniformis* or *Bacillus subtilis.*

18. The production method according to claim 17, wherein the protease is one or more selected from the group consisting of serine protease derived from *Bacillus licheniformis* and metalloprotease derived from *Bacillus subtilis.*

19. The production method according to any one of claims 13 to 16, wherein the protease is a protease derived from *Aspergillus oryzae.*

20. The production method according to claim 19, wherein the protease is a mixture of serine protease, aspartic protease and metalloprotease derived from *Aspergillus oryzae.*

21. The production method according to any one of claims 13 to 16, wherein the protease is one or more selected from the group consisting of the following (a) to (h):
(a) aspartic protease derived from *Aspergillus oryzae;*
(b) a mixture of aspartic protease and serine protease derived from *Aspergillus oryzae;*
(c) serine protease derived from *GeoBacillus stearothermophilus;*
(d) metalloprotease derived from *Bacillus amyloliquefaciens;*
(e) serine protease derived from *Aspergillus melleus;*
(f) pepsin;
(g) papain;
(h) bromelain.
